# DEMANDE DE BREVET EUROPEEN

(11) **EP 4 717 199 A1**
(43) Date de publication de la demande: **01.04.2026**
(21) Numéro de dépôt: 24202385.1
(22) Date de dépôt: 25.09.2024
(51) Int. Cl.: A61B 17/70, A61B 17/88, A61F 2/46, A61M 25/10

(54) **IMPLANT OSSEUX EXPANSIBLE DE CHIRURGIE ORTHOPÉDIQUE HUMAINE ET SYSTÈME ORTHOPÉDIQUE**

(71) Demandeur: Lock-In VCF SA, 1180 Rolle (CH)
(72) Inventeur: Lacaze, Guillaume, 1278 La Rippe (CH)
(74) Mandataire: Gsmart

(57) **Abrégé**

1. La présente invention concerne un Implant osseux expansible de chirurgie orthopédique humaine, un Système orthopédique et un Procédé de fabrication de l'implant, pour la restauration de volume et/ou géométrie d'un os, par une expansion entre une configuration repliée et une configuration déployée, au moins deux faces de l'implant comportant chacune au moins un plateau (13, 14, 15) pour le contact avec les tissus osseux, chacun des plateaux étant supportés par au moins deux bras de support (131, 141, 151) chacun, par l'intermédiaire d'une charnière sur l'axe central (3) et d'une charnière sous le plateau (13, 14, 15) respectif de chacun desdits bras de support (131, 141, 151) ;
caractérisé en ce que :
- une douille ou bague d'expansion (20) disposée dans le même axe que ledit axe central (3) ;
- au moins deux bras d'expansion (132, 142, 152) articulés sur l'un des plateaux (13, 14, 15) et sur ladite douille ou bague d'expansion (20) ;
- un éloignement entre ladite douille (3) et ledit axe central (3), exerçant une traction sur les plateaux (13, 14, 15), par l'intermédiaire des bras d'expansion (132, 142, 152), ce qui engendre un pivotement desdits bras de support (131, 141, 151) provoquant l'éloignement des plateaux (13, 14, 15) par rapport à l'axe central (3), de manière à résulter en une expansion contrôlée de l'implant (1) ;
et en ce qu'il comporte une enveloppe renfermant ledit implant depuis l'extrémité proximale (11) jusqu'à l'extrémité distale (12) et en ce que :
- ladite enveloppe est formée par une feuille (10) en alliage biocompatible de métaux, refermée sur elle-même de manière étanche, pliées avec des plis enroulés autour de l'axe longitudinal (L) ;
- ladite feuille (10) est déformable plastiquement pour permettre l'expansion de l'implant de la configuration repliée à la configuration déployée en formant une enveloppe étanche enfermant l'implant et permettant d'éviter les fuites lors de l'injection d'un fluide à l'intérieur de l'implant (1) et l'enveloppe.

## Description

La présente demande se rapporte au domaine de la chirurgie, en particulier la chirurgie orthopédique humaine et notamment du traitement d'une structure osseuse affaissée, par une restauration du volume (ou redressement) de cette structure osseuse. La présente demande concerne en particulier un implant et son procédé de fabrication, ainsi qu'un système pour la restauration de structure osseuse, notamment dans le rachis pour le traitement (souvent appelé « réduction ») des fractures de compression, notamment vertébrales (VCF pour l'anglais « Vertébral Compression Fracture »).

Dans ce domaine, le problème de la restauration du volume de structure osseuse qui s'est affaissée est bien connu et la littérature abonde de solutions utilisant des implants expansibles capables de passer d'une configuration repliée à une configuration déployée pour restaurer la hauteur de la structure osseuse, de préférence en combinaison avec une injection de ciment de substitution osseuse, dit ciment (ou ciment osseux). De nombreux ciments sont connus et ils présentent tous l'avantage d'être injectables à l'état liquide ou visqueux pendant une certaine durée, puis de durcir (par polymérisation) à l'intérieur de la structure osseuse pour la stabiliser.

Un problème majeur dans ce domaine concerne l'expansion de l'implant pour restaurer une hauteur au tissu osseux lésé. De nombreuses solutions sont connues de l'art antérieur, comme notamment les demandes de brevet EP3086729, US 11540926, EP3747385, EP2572680, EP3958752, EP2693967, EP2405835, US9579130, EP4216836, WO2023122005, WO2022162418, EP3843668 ou US10945861 mais ces solutions présentent divers problèmes de difficulté de manipulation pour le déploiement, de stabilité et de fiabilité une fois déployés. De plus, ces solutions connues s'accompagnent généralement d'une injection de ciment osseux mais ne fournissent aucun enseignement relatif à la fuite de ciment en dehors de l'implant, alors qu'une telle fuite peut être préjudiciable pour les tissus environnants, voire même l'organisme tout entier si les substances chimiques du ciment envahissent le système sanguin. En effet, le ciment comporte en général une ou plusieurs substances chimiques polymérisables, par exemple comme le poly(méthacrylate de méthyle) (PMMA, pour l'anglais poly-methyl-methacrylate) et éventuellement des additifs. De plus, la température atteinte lors de la polymérisation du ciment n'est pas inoffensive car elle est généralement supérieure à 60°.

Il est connu de l'art antérieur, notamment des document EP1308134, US9510877, US8936627 ou EP2467099, des dispositifs de redressement et stabilisation (ou réduction de fracture d'os), notamment du rachis sous la forme de stent, ou sous forme de ballons ou de sacs gonflables poreux comme dans les documents EP1408888 ou EP1379185, éventuellement équipé de plateaux de support comme dans le document US20060100706. De nombreux documents proposent ce genre de stent, c'est-à-dire d'endoprothèse déformable similaire aux endoprothèses, extenseurs ou tuteurs vasculaires, qui sont généralement sous la forme d'un corps tubulaire maillé, le plus souvent métallique et déformable par l'introduction d'un ballon gonflable pour dilater le corps en écartant les mailles, le ballon étant ensuite retiré pour permettre une injection de ciment, qui durcit et forme ainsi une structure de redressement et de stabilisation. Cependant, ces dispositifs présentent l'inconvénient de nécessiter une implantation en deux temps avec le gonflage du ballon puis l'injection de ciment, ce qui ralentit et complexifie l'opération mais présente également un risque d'affaissement du dispositif entre le dégonflage du ballon et le remplissage du stent par le ciment. D'autre part, ces solutions présentent l'inconvénient de ne pas répondre au problème majeur des fuites de ciment.

Il est également connu, notamment des documents EP1938765, EP2351539 ou WO200434924, des solutions utilisant des implants à structure maillée, en métal à mémoire de forme, qui est contrainte dans une forme repliée pour l'insertion dans le tissu osseux et capable de s'expandre spontanément, lors du relâchement de la contrainte et/ou sous l'effet de la chaleur. Ces solutions présentent l'inconvénient d'une utilisation d'alliages coûteux et une fabrication complexe pour obtenir une mémoire de forme adéquate qui convienne à l'implantation visée, ce qui implique un surcoût en multipliant le nombre d'implants différents nécessaires pour couvrir les différents cas pathologiques, notamment par l'amplitude de la déformation dont est capable le matériau à mémoire de forme. De plus, la force exercée par le retour du métal à sa forme non contrainte n'est souvent pas suffisante pour redresser correctement la structure osseuse qui s'est affaissée, ou du moins limitative. D'autre part, ces solutions présentent également l'inconvénient de ne pas répondre au problème majeur des fuites de ciment.

Il est également connu de l'art antérieur, notamment des documents EP2405835, US9579130, EP2572680 ou EP1956990 des solutions utilisant des implants expansibles utilisant un mécanisme de levier, à la manière d'un cric de voiture (« jack » en anglais) pour restaurer la structure osseuse à une hauteur déterminée. Ces solutions présentent l'avantage de ne pas risquer d'affaissement contrairement à un sent déployés par un ballon qui est retiré avant l'injection de ciment, mais ils présentent également l'inconvénient d'une implantation en deux temps et que les dimensions de la surface de support pour exercer la force d'expansion sur le tissu osseux est limitée, en comparaison avec les stents notamment. D'autre part, ils présentent également les inconvénients d'être coûteux et de ne pas répondre non plus au problème majeur des fuites de ciment.

Le problème de fuite de ciment a déjà été identifié, notamment dans les documents EP1408888, EP1509175 ou WO200394805 qui expriment l'intérêt que présenterait un implant déformable peu perméable ou imperméable pour limiter ou éviter les fuites de ciment. Ces documents envisagent de nombreuses solutions pour un implant expansible, en métal ou en polymère, qui pourrait être soit souple et flexible comme une membrane ou un tissu, voire élastique, soit semi-rigide (« conformable ») ou rigide, soit en matériau à mémoire de forme, avec une paroi continue ou fenestrée (i.e., maillée) et qui pourrait être poreux ou non poreux. Cependant, toutes ces hypothèses décrites dans ces documents définissent surtout des méthodes envisageables de traitement et des objectifs à atteindre, sans fournir de réel enseignement en ce qui concerne les caractéristiques techniques, ou l'agencement structurel des implants, ni sur la façon d'obtenir de tels implants et ainsi mettre en oeuvre ces méthodes. Ces propositions présentent donc un problème majeur de faisabilité technique.

D'autre part, un problème qui n'est pas identifié dans l'art antérieur concerne du site d'injection du ciment et la répartition des forces exercées sur les tissus osseux pour les redresser. En effet, l'imperméabilité d'un implant permet d'éviter les fuites de ciment, mais la nature de la membrane imperméable et ses caractéristiques techniques comme ses propriétés physico-chimiques et mécaniques influent sur sa capacité à se déployer sans se rompre et à redresser la structure osseuse. Ainsi, une membrane élastique présente l'inconvénient de se déformer excessivement dans les zones à faible densité et d'avoir ainsi une capacité limitée à restaurer une hauteur, avec en plus le risque de se rompre aux endroits où son élasticité maximale est dépassée à cause de cette déformation incontrôlée. Une membrane semi-rigide est donc préférable, mais ce problème de déformation implique également un problème de formes de l'implant, en configuration repliée et surtout en configuration déployée. En effet, la forme de l'implant déployé délimite le site d'injection du ciment et la maîtrise de ce site est importante pour la répartition des forces conduisant à remplir les zones à faible densité tout en redressant la structure (notamment en hauteur), ce qui impacte la réussite de l'opération. Ainsi, on comprend que la fourniture d'un implant répondant à l'ensemble de ces problèmes s'accompagne d'un problème de faisabilité technique et donc de fabrication.

D'autres problèmes récurrents en chirurgie orthopédique concernent l'invasivité (c'est-à-dire le but de faire une incision et des lésions les moins étendues possibles) mais également le ratio de déploiement afin d'obtenir un implant déployé qui comble le plus grand volume possible tout en ayant été introduit par un passage le plus petit possible. D'autre part, ce ratio de déploiement va impacter la répartition des forces pour redresser les vertèbres : si on est trop déformable, la pression d'injection du ciment va plutôt déformer la poche au lieu de restaurer la hauteur.

Un problème complémentaire à celui du déploiement concerne le repliement qui n'est généralement pas possible dans les implants de l'art antérieur. La maîtrise du repliement permet la maîtrise du déploiement et donc du site d'injection avec une répartition homogène du ciment et de la pression pour remplir l'espace à combler suite à l'affaissement. Homothétie parfaite s'adaptant à la fracture en respectant la forme de l'os à l'intérieur de la fracture.

Dans ce contexte, on comprend qu'il persiste dans le domaine un problème technique concernant la restauration de structure osseuse (redressement ou réduction de fracture ou augmentation de volume après affaissement) grâce à un implant expansible (déployable) qui soit capable d'expandre les tissus osseux affaissés et suffisamment imperméable pour éviter ou limiter la fuite de ciment en-dehors de l'implant avec une maîtrise du site d'injection.

Enfin, un problème principal qui persiste dans le domaine concerne la faisabilité technique de la fabrication des implants proposés dans l'art antérieur. Il est par exemple connu le document WO200394805 décrit de nombreuses méthodes d'administration de substances et notamment de ciment osseux, avec de nombreuses variantes envisagées pour un implant expansible, en métal ou en polymère, qui pourrait être soit souple et flexible comme une membrane ou un tissu, soit semi-conformable ou rigide, soit en matériau à mémoire de forme, avec une paroi continue ou fenestrée (i.e., maillée) et qui pourrait être poreux ou non poreux. Cependant, ce document ne décrit que des méthodes envisageables de traitement, mais ne fournit aucun enseignement en ce qui concerne les caractéristiques techniques, ou l'agencement structurel de ces nombreux implants hypothétiques utilisés pour ces méthodes envisagées, ni sur la façon d'obtenir de tels implants et ainsi réellement mettre en oeuvre ces méthodes. Ces propositions présentent donc un problème majeur de faisabilité technique et définissent plus des buts à atteindre que des moyens d'y parvenir. De plus, même si de nombreux objectifs ont été détaillés dans la littérature, de nombreux implants proposés pour atteindre ces objectifs n'ont jamais vu le jour à cause de problèmes de fabrication. Pour répondre au problème de fabrication, il est nécessaire de prendre en compte les problèmes liés à au souhait de compacter / replier un « sac » (ballon/poche) en matériau rigide et imperméable pour :
- passer dans un conduit cylindrique ;
- rendre possible l'expansion sans rupture de la poche, malgré la rigidité et la différence de volume souhaité entre le volume replié et le volume déployé ;
- Maîtriser le volume et la répartition des forces d'expansion sur l'os.

Dans ce contexte, un but de la présente invention est de pallier au moins certains inconvénients de l'art antérieur en proposant un implant de restauration de structure osseuses affaissées fiable et simple à manipuler et implanter.

Ce but est atteint par un implant osseux expansible de chirurgie orthopédique humaine pour la restauration de volume et/ou géométrie d'un os, par une expansion entre une configuration repliée et une configuration déployée, ledit implant comportant un axe central et s'étendant selon un axe longitudinal entre une extrémité proximale apte à coopérer avec un instrument d'implantation pour tenir l'implant et une extrémité distale destinée à être insérée en premier dans l'os, au moins deux faces, par exemple supérieure et inférieure, de l'implant comportant chacune au moins un plateau pour le contact avec les tissus osseux, chacun des plateaux étant supportés par au moins deux bras de support chacun, par l'intermédiaire d'une charnière sur l'axe central et d'une charnière sous le plateau respectif de chacun desdits bras de support ; caractérisé en ce que :
- une douille ou bague d'expansion disposée dans le même axe que ledit axe central ;
- au moins deux bras d'expansion comportent chacun une charnière les reliant à l'une des extrémités de l'un des plateaux et une charnière les reliant à ladite douille ou bague d'expansion ;
- ladite douille ou bague d'expansion et l'extrémité proximale de l'axe central sont aptes à coopérer, respectivement ou inversement, avec un tube creux de préhension de l'implant d'un instrument d'implantation et avec une tige d'expansion dudit instrument ;
- ladite tige d'expansion est apte à coulisser à l'intérieur dudit tube creux, de façon à permettre de provoquer un éloignement entre ladite douille et ledit axe central, exerçant une traction sur les plateaux, par l'intermédiaire des bras d'expansion, ce qui engendre un pivotement desdits bras de support provoquant l'éloignement des plateaux par rapport à l'axe central, de manière à résulter en une expansion contrôlée de l'implant entre ladite configuration repliée et ladite configuration déployée.

Selon une autre particularité, c'est ladite bague d'expansion qui est apte à coopérer avec un tube creux de préhension de l'implant dudit instrument d'implantation, en laissant passer la tige d'expansion à travers la bague, tandis que l'axe central est apte à coopérer avec ladite tige d'expansion dudit instrument, de sorte qu'une poussée exercée sur ladite tige d'expansion coulissant à l'intérieur dudit tube creux provoque l'éloignement dudit axe central par rapport à ladite bague, résultant en une expansion de l'implant contrôlée en fonction de la force de poussée exercée sur la tige d'expansion.

Selon une autre particularité, c'est l'extrémité proximale de l'axe central qui est apte à coopérer avec ledit tube creux de préhension de l'implant dudit instrument d'implantation, tandis que ladite douille ou bague d'expansion est apte à coopérer avec ladite tige d'expansion dudit instrument, de sorte qu'une poussée exercée sur ladite tige d'expansion coulissant à l'intérieur dudit tube creux provoque l'éloignement de ladite douille ou bague d'expansion par rapport audit axe central, résultant en une expansion de l'implant contrôlée en fonction de la force de poussée exercée sur la tige d'expansion.

Selon une autre particularité, l'axe central comporte un conduit apte à coopérer avec ladite tige d'expansion qui est, d'une part, creuse et munie d'au moins une ouverture au niveau de son extrémité distale et, d'autre part, connectable à un instrument d'injection de fluide pour acheminer au moins un fluide dans le site d'implantation de l'implant via l'axe central, de préférence muni d'ouvertures pour faire déboucher le fluide dans la longueur de l'axe central.

Selon une autre particularité, les bras de support des plateaux comportent deux bras reliés respectivement à proximité des extrémités proximale et distale de leur plateau respectif, pour fournir un support sur toute la longueur des plateaux et limiter leurs risques de cintrage ou fluage.

Selon une autre particularité, au moins un bras de support central supplémentaire est relié entre une portion centrale de l'axe central et une portion centrale des plateaux, avec des charnières aux deux extrémités du bras de support pour son pivotement.

Selon une autre particularité, l'implant comporte deux plateaux disposés de part et d'autre de l'axe central pour fournir un appui contre des tissus osseux lésés de part et d'autre de l'implant, par exemple pour restaurer une hauteur ou une largeur.

Selon une autre particularité, l'implant comporte au moins un plateau additionnel, la répartition des plateaux autour de l'axe central variant en fonction de leur nombre et/ou des besoins en termes de traitement chirurgical, avec de préférence une équi-répartition angulaire radialement par rapport à l'axe central, pour exercer une compression homogène sur les tissus osseux à la périphérie de l'implant.

Selon une autre particularité, la distance entre un pli synforme et le pli antiforme suivant est plus longue que la distance entre un pli antiforme et le pli synforme suivant, pour faciliter l'enroulement des plis autour de l'axe longitudinal de l'implant en configuration repliée.

Selon une autre particularité, ledit manchon d'étanchéité prolonge ladite extrémité proximale, parallèlement à l'axe longitudinal, jusqu'à une distance qui est supérieure ou égale à celle à laquelle s'étendent les plateaux depuis le centre de l'implant

Selon une autre particularité, ledit manchon d'étanchéité possède une ouverture traversante dont le diamètre est supérieur ou égal à celui de l'ouverture du manchon de coulissement, de sorte que ces deux manchons d'étanchéité et de coulissement fournissent une entrée vers l'intérieur du corps creux de l'implant depuis un conduit d'un instrument d'implantation tenant l'implant au niveau de l'extrémité proximale, apte à acheminer un fluide à injecter dans ledit implant.

Selon une autre particularité, l'implant comporte, en position déployée, une portion médiane entre ses deux extrémités qui possède une forme de cylindre généralisé de longueur supérieure ou égale à celle des plateaux, avec une persistance éventuelle et partielle desdits plis, ladite portion médiane se prolongeant, du côté de l'extrémité proximale, par une portion tronconique reliant la portion médiane au manchon et, du côté de l'extrémité distale, par une portion tronconique reliant la portion médiane à la douille, les portions tronconiques présentant une persistance permanente d'au moins une partie de plis couchés et enroulés à proximité des extrémités proximale et distale.

Selon une autre particularité, ladite feuille est déformable plastiquement également de la configuration repliée à la configuration déployée, notamment grâce à la persistance des plis couchés et enroulés aux extrémités proximale et distale, facilitant la réversibilité de l'expansion.

Selon une autre particularité, ledit axe central est apte à coopérer avec et/ou se prolonge au-delà de l'extrémité distale d'un instrument d'implantation au niveau de l'extrémité proximale de l'implant et possédant un conduit intérieur en communication avec un conduit ménagé dans ledit axe central débouchant dans l'espace ménagé par l'écartement des plateaux, via au moins une ouverture permettant l'injection dudit fluide dans l'implant.

Selon une autre particularité, ladite feuille est solidarisée à l'extrémité proximale par une soudure figeant l'extrémité proximale des plis couchés et enroulés contre la paroi extérieure dudit manchon de coulissement et/ou solidarisée à l'extrémité distale par une soudure figeant l'extrémité distale des plis couchés et enroulés contre la paroi extérieure de ladite douille de traction.

Selon une autre particularité, ladite feuille est comprimée autour du manchon de l'extrémité proximale et/ou autour de la douille de l'extrémité distale par une bague de compression maintenant les plis couchés et enroulés contre la paroi extérieure dudit manchon et/ou de ladite douille.

Selon une autre particularité, les plis sont, au moins dans la configuration repliée, parallèles à l'axe longitudinal.

Selon une autre particularité, la feuille comporte également au moins une paire de plis (un pli synforme et un pli antiforme) d'axe non parallèle à l'axe longitudinal, de préférence perpendiculaire pour une expansion également en longueur de l'implant ou oblique pour une expansion incurvée de l'implant.

Selon une autre particularité, le nombre de paires de plis est compris entre 3 et 16, généralement 4 à 12, de préférence de l'ordre de 8.

Selon une autre particularité, la feuille possède une épaisseur comprise entre 3 et 100 microns, généralement entre 6 et 50 et de préférence 10 et 30 microns.

Selon une autre particularité, la feuille est en alliage de titane.

Selon une autre particularité, la distance entre les plis est variable d'une face latérale à l'autre de l'implant, de sorte que la forme de l'implant en configuration déployée soit incurvée et/ou asymétrique transversalement à l'axe longitudinal.

Un autre but de la présente demande est de pallier au moins une partie des inconvénients de l'art antérieur en proposer un système d'intervention chirurgicale facile à utiliser et permettant une stabilisation efficace des tissus osseux

Ce but est atteint par un système de traitement orthopédique de tissu osseux lésé comprenant un ciment de substitution osseuse et au moins un instrument d'implantation et d'injection de ciment dans l'implant, caractérisé en ce qu'il comporte un implant selon l'un des modes de réalisation décrit dans la présente demande.

Selon une autre particularité, l'instrument d'implantation et d'injection de ciment comporte des moyens de contrôle de la pression et/ou de l'aspiration du ciment pour replier l'implant en configuration repliée si nécessaire.

Selon une autre particularité, l'instrument d'implantation est distinct mais complémentaire de l'instrument d'injection dont le canal d'injection du ciment traverse un canal à l'intérieur de la tige de l'instrument d'implantation tenant l'extrémité proximale de l'implant grâce à son extrémité distale.

D'autres particularités et avantages de la présente invention apparaîtront plus clairement à la lecture de la description de divers modes de réalisation ci-après, faite en référence aux dessins annexés, dans lesquels :

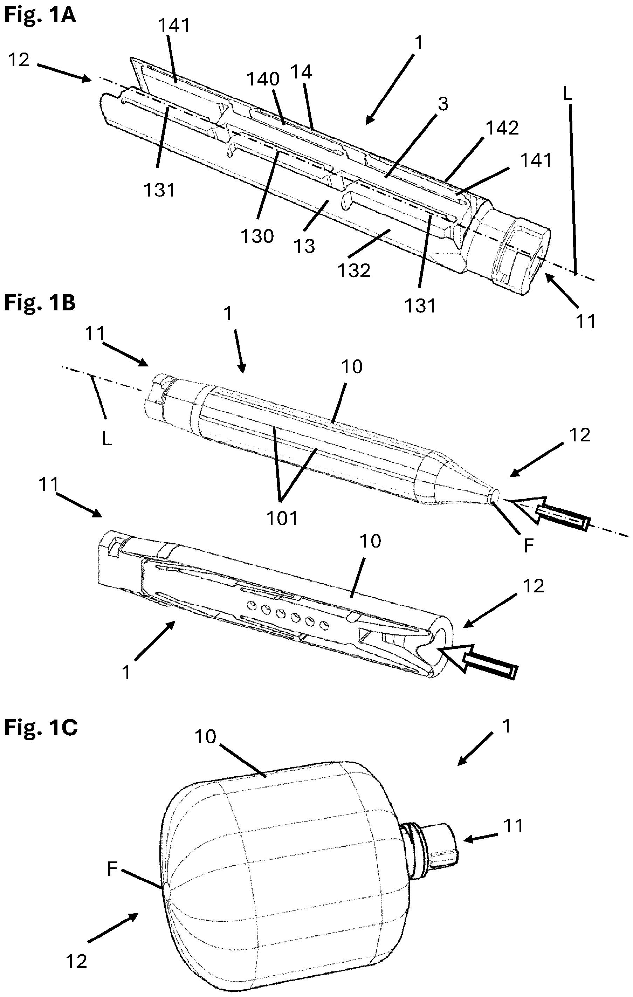

La figure 1A représente une vue en perspective d'un implant expansible selon un certain mode de réalisation dépourvue de son enveloppe, la figure 1B représente une vue en perspective d'un implant muni de son enveloppe avant le repliement de l'extrémité distal de l'enveloppe et après repliement à cette extrémité distale et la figure 1C représente une vue en perspective de l'implant de la figure 1B mais en configuration déployée
La figure 2A représente une vue en perspective d'un implant du type de celui de la figure 1C mais avec l'enveloppe coupée dans sa partie médiane, la figure 2B représente une vue de profil d'un implant expansible en configuration déployée munie de double bras supports et possédant un mécanisme d'auto-verrouillage et la figure 2C représente un détail de l'extrémité proximale d'un implant selon différents de réalisation ;

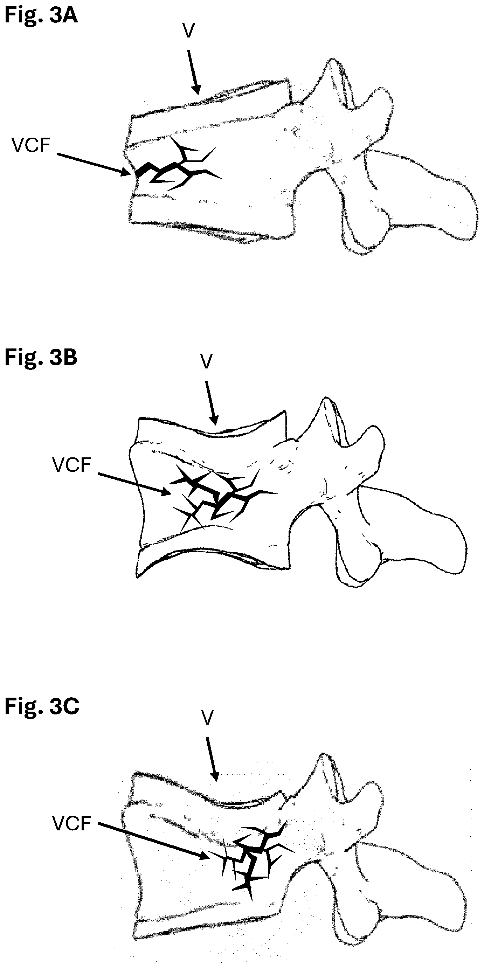

Les figures 3A, 3B et 3C représentent des vues de profil de vertèbres ayant subi des fractures de compression vertébrale (VCF) respectivement au niveau antérieur médian et postérieur ;
Les figures 4A, 4B, 4C et 4D représentent des vues de profil de quatre implants expansibles en configuration repliée selon différents modes de réalisation, avec des bras de supports de différentes longueurs ;
Les figures 5A, 5B, 5C et 5D représentent des vues de profil des implants respectivement des figures 4A, 4B, 4C et 4D mais en configuration déployée avec leurs plateaux non parallèles ;

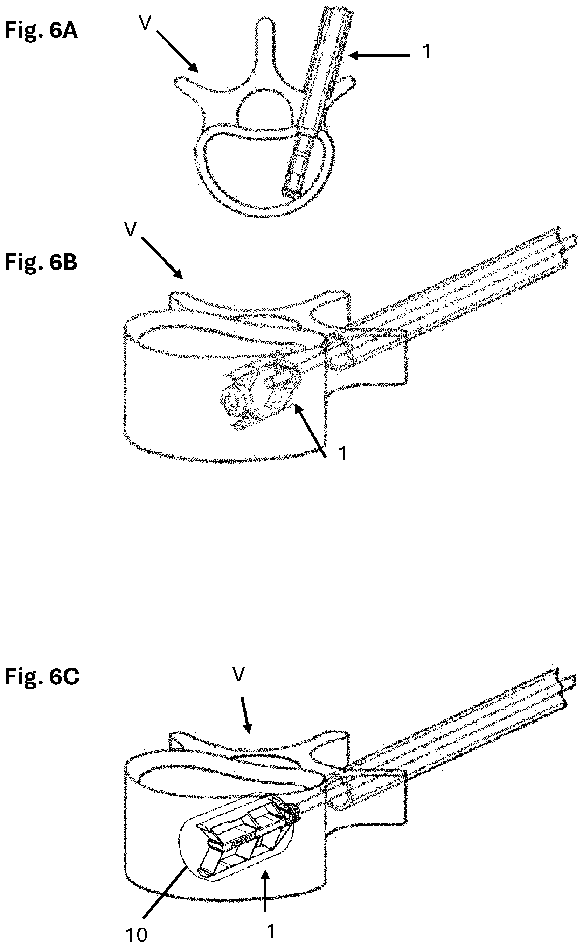

La figure 6A représente une vue de dessus d'une vertèbre dans laquelle est implanté un implant selon divers modes de réalisation, la figure 6B représente une vue de perspective d'une vertèbre dans laquelle est implanté un implant de l'art antérieur et la figure 6C représente une vue en perspective d'une vertèbre dans laquelle est implanté un implant selon certains modes de réalisation ;

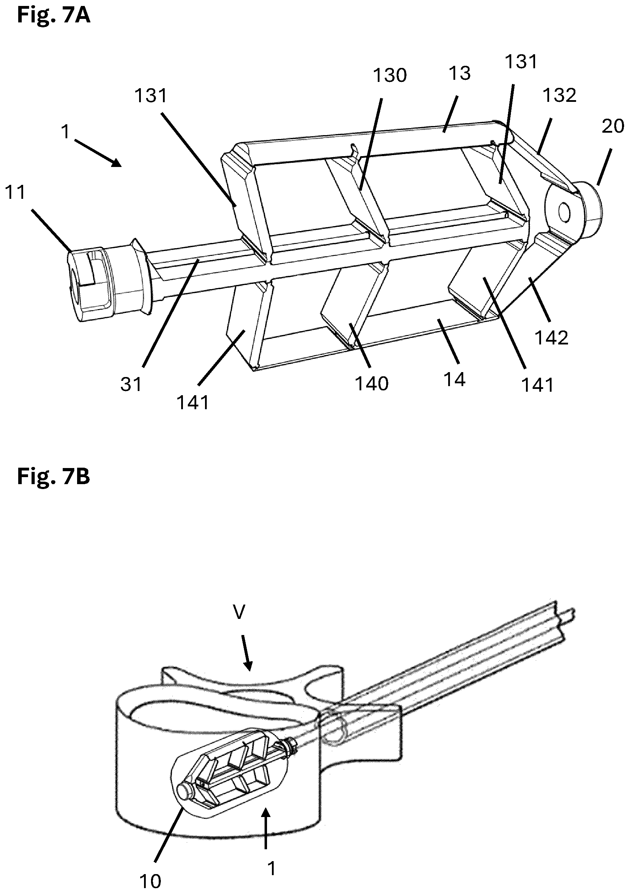

La figure 7A représente une vue en perspective d'un implant expansible selon un mode de certains de réalisation inversée et la figure 7 b représente une vue perspective d'une vertèbre dans laquelle est implanté, à l'aide d'un instrument, l'implant de la figure 7A ;

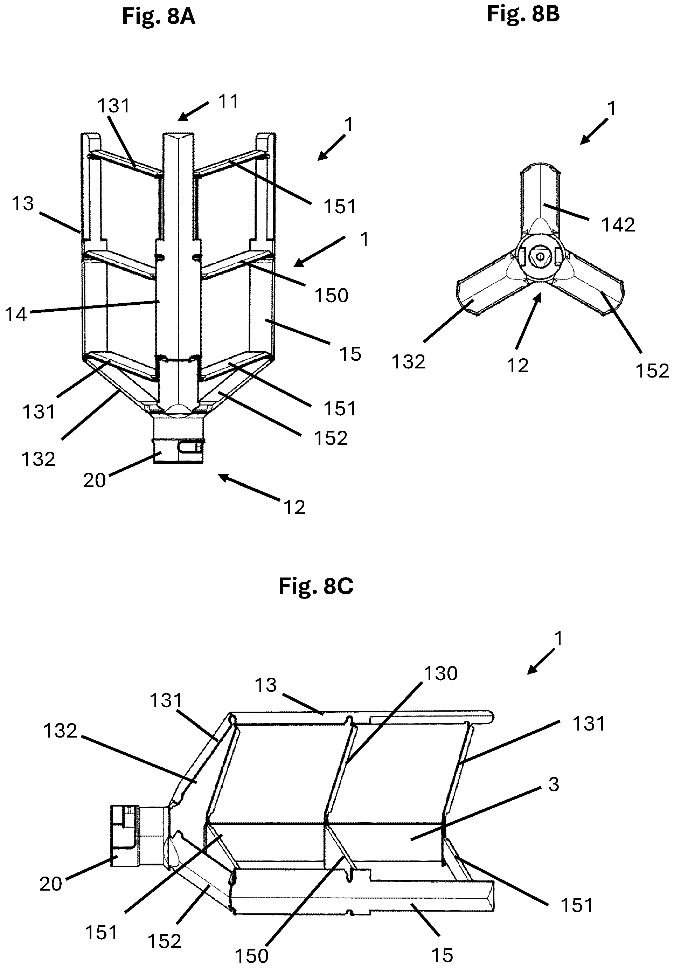

Les figures 8A, 8B et 8C représentent respectivement et une vue de dessus, une vue de face et une vue de profil d'un implant expansible à trois plateaux selon certains modes de réalisation ;

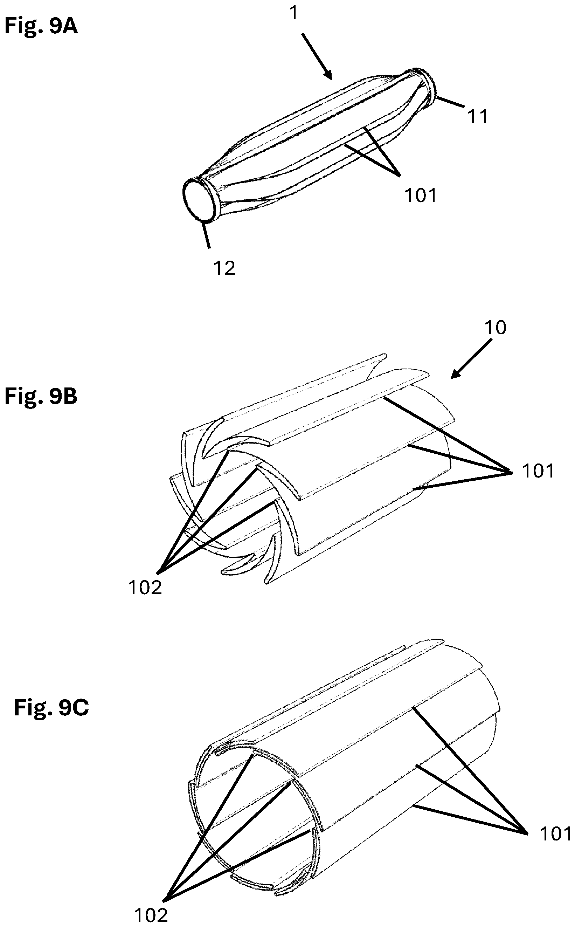

La figure 9A représente une vue en perspective d'un implant expansible en configuration semi-déployée, selon certains modes de réalisation, la figure 9B représente une vue en perspective d'une feuille utilisée pour la fabrication d'un implant expansible selon certains modes de réalisation en configuration semi-repliée et la figure 9C représente une vue en perspective de cette même feuille en configuration repliée.

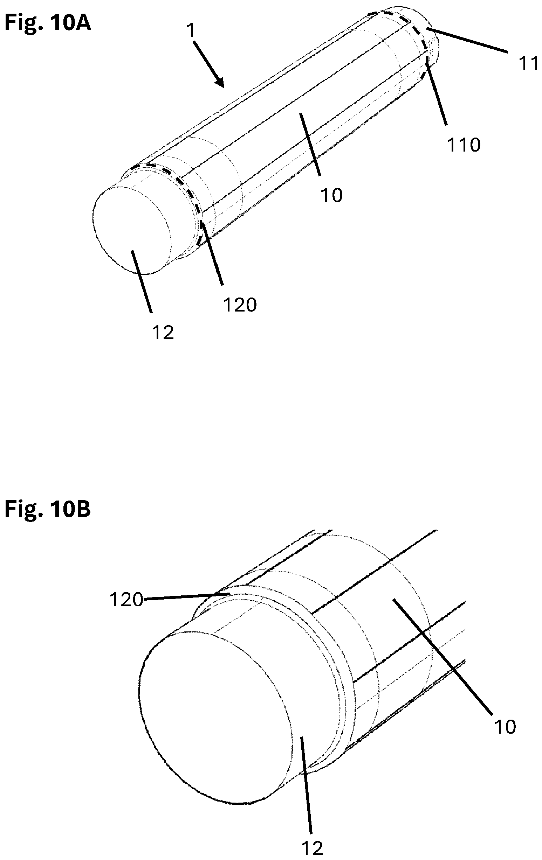

La figure 10A représente une vue en perspective d'un implant expansible en configuration repliée avec les lignes de soudure aux extrémités proximales et distale et la figure 10B représente un agrandissement de la figure 10A au niveau de l'extrémité distale.

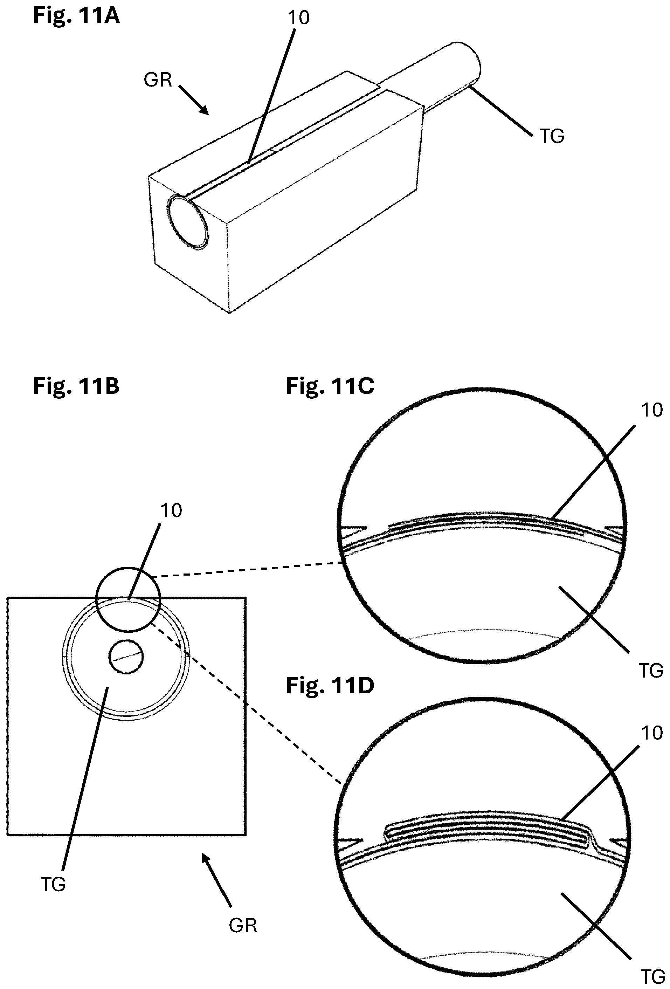

La figure 11A représente une vue en perspective d'un outil de guidage du repliement d'une feuille d'un implant expansible selon certains modes de réalisation guidée à l'aide d'un tube de guidage ;
La figure 12A représente une vue en perspective d'un outil de pré-pliage de feuilles pour un plan selon le divers mode de réalisation avec une platine de pré-pliage ;
La figure 13A représente un agrandissement de la figure 12, les figures 13B, 13C et 13D représentent les vues de dessus de différents modes de réalisation de l'outil de pré-pliage avec sa tige étoilée et la feuille de l'implant glissée autour ;
La figure 14A représente une vue en perspective d'une feuille pré-plié à l'aide d'une tige étoilée telle que celle de la figure 13D et la figure 14B représente cette même feuille repliée sur elle-même, selon certains modes de réalisation la figure 14A représente une vue de dessus d'une vertèbre dans laquelle est implantée un implant selon divers modes de réalisation ;
La figure 15A représente une vue en perspective d'un implant expansible en configuration déployée et munie d'un verrou maintenant l'implant dans sa configuration déployée, la figure 15B représente un détail de l'extrémité proximale d'un implant du type de figure 15A avec le verrou à l'extérieur de l'implant selon certains modes de réalisation et la figure 15C représente un détail d'une de l'extrémité proximale d'un implant selon certaines réalisations avec un autre type de verrou à l'extérieur de l'implant.

La présente demande concerne un implant et un système de chirurgie orthopédique pour le traitement d'os fracturé et de tissus osseux en général, ainsi qu'un procédé de fabrication de l'implant. L'implant osseux est de préférence un implant Rachidien, et en particulier vertébral ou même en fait intravertébral, mais d'autres utilisations sont envisageables ailleurs dans le rachis (épines intervertébrales) ou dans d'autres structures osseuses où il est nécessaire de combler un espace laissé résultant d'une fracture (dont les causes peuvent être diverses, même si elles impliquent généralement une diminution de densité osseuse). Ainsi, les fractures vertébrales de compression (VCF pour l'anglais Vertébral Compression Fracture) sont une application favorite mais ne sont pas les seules à pouvoir être traitées grâce à la présente invention et l'homme de métier appréciera les possibilités offertes sans nécessiter plus de détails ici. Comme autre os, on peut citer le fémur ou l'humérus (tête) par exemple en cas de risque d'effondrement. Certains modes de réalisation comportant plus de deux plateaux peuvent notamment servir plus efficacement au traitement d'os longs de ce type en répartissant les forces d'expansion sur plus deux surfaces, ce qui fournit une meilleure stabilité quel que soit le type d'os.

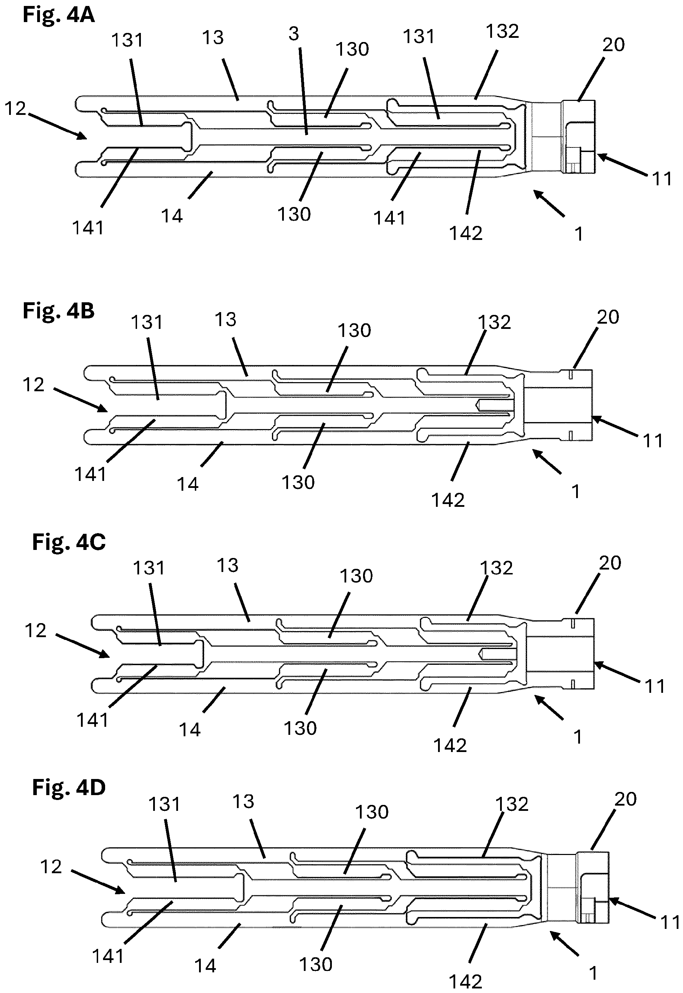

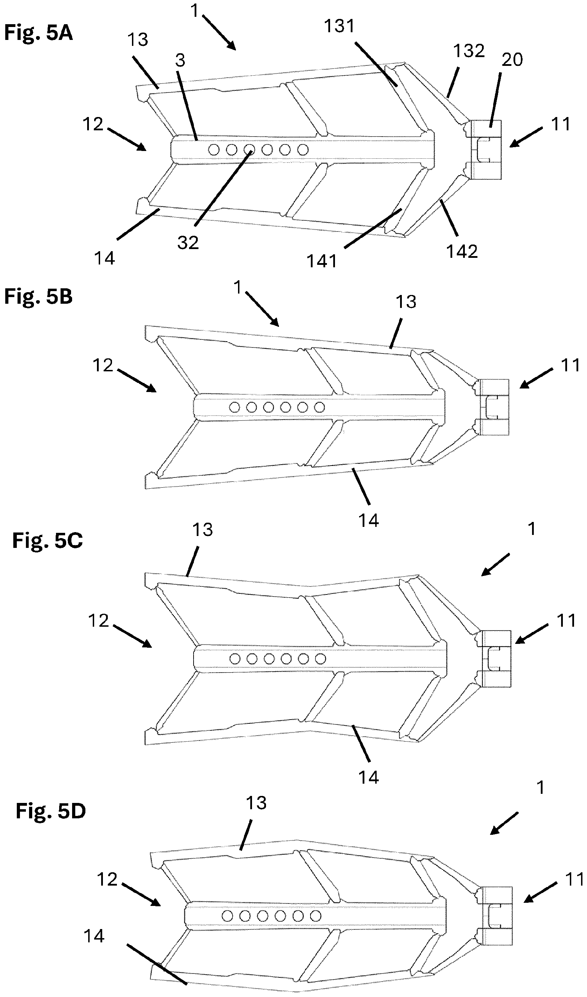

D'autre part, dans divers modes de réalisation qui ne concernent pas nécessairement des os longs, l'implant comporte des bras support au niveau distal qui ont une longueur différente de celle des bras support au niveau proximal et/ou des bras de support central, de sorte que l'implant en configuration déployée possède des plateaux qui ne sont pas parallèles entre eux. Des exemples illustratifs et non limitatifs de tels modes de réalisation sont représentés sur les figures 4A, 4B, 4C et 4D en configurations repliées et sur les figures 5A, 5B, 5C et 5D correspondant à leurs configurations déployées respectives. On comprend que des bras plus longs au niveau proximal (Fig. 4A et 5A) permet d'obtenir des plateaux inclinés en direction de l'extrémité distale, tandis que des bras plus longs au niveau distal (Fig. 4B et 5B) permet d'obtenir des plateaux inclinés en direction de l'extrémité proximale. De plus, il est possible de prévoir des plateaux qui ne restent pas plans à la fin de l'expansion, ce qui représente un net avantage de l'implant qui peut alors épouser les formes anatomiques des os où ils sont implantés. Ainsi, une forme concave ou bi-concave (i.e., concave sur les deux faces des plateaux) de l'implant déployée peut être obtenue avec des bras centraux ((130, 140) plus courts que les bras support (131, 141) comme sur les figures 4C et 5C, tandis qu'une forme convexe (ou bi-convexe : i.e., convexe sur les deux faces des plateaux) comme sur les figures 4D et 5D.

Certains modes de réalisation prévoient l'injection d'un fluide (e.g., « ciment osseux », généralement à base d'un polymère comme le PMMA par exemple et bien connu de l'homme de métier, de sorte qu'aucun détail sur le ciment ne sera fourni ici). Ainsi, l'implant une fois positionné peut notamment être stabilisé grâce une telle injection de ciment. Cependant, comme les fuites de ciment restent un problème majeur dans le domaine, divers modes de réalisation proposent de contenir le ciment dans une enveloppe étanche dont le volume après injection peut être maîtrisé grâce à la structure et le matériau de l'enveloppe, en fonction de la pression injectée (et de la configuration des tissus osseux préférentiellement évaluée à l'avance, comme généralement pratiqué dans le domaine). L'étanchéité est bien entendu relative et ce terme n'est pas limitatif non plus, puisque le niveau d'étanchéité est en fait adapté à la viscosité du ciment au moment où on l'injecte. Certains modes de réalisation permettent en particulier un gonflement homothétique de l'enveloppe grâce à la souplesse (relative) de la feuille (10) de matériau métallique biocompatible. Ce matériau est en général un alliage de titane obtenu sous forme de feuille très fine, de préférence par lamination pour un état de surface et une épaisseur maîtrisés, notamment une épaisseur comprise entre 3 et 100 microns, généralement entre 6 et 50 et de préférence 10 et 30 microns. Cette feuille est capable de déformation plastique réversible un nombre de fois satisfaisant pour l'application visée puisqu'elle offre notamment la possibilité de rétracter l'enveloppe formée par la feuille en cas de problème (biocompatibilité et résistance au déchirement). En effet, en général, le pilotage du dosage de ciment permet de surveiller les quinze minutes de polymérisation pendant lesquelles il est possible de rétracter l'enveloppe et aspirer le ciment. D'autre part, par l'injection de ciment, le gonflement de l'enveloppe, l'implant remplit les espaces dans les tissus lésés en fonction des forces de compression et de résistance osseuse par rapport à la pression hydraulique fournie lors de l'injection de ciment. A partir d'une telle feuille, il est nécessaire d'obtenir une structure fermée, ce qui impose déjà de refermer la feuille sur elle-même et de la verrouiller en position. Pour cela, une soudure (ou un collage ou une brasure, ces termes n'étant pas limitatifs ici) peut être réalisée entre deux bords qui se superposent ou sur des bords avec des revers enchevêtrés, pour faciliter et solidifier la soudure. Certains modes de réalisation prévoient donc une fermeture par soudure depuis l'extérieur, simplifié et plus solide grâce à la superposition de couches au niveau de ces plis complémentaires.

Le terme « solidarisé » signifie ici que deux éléments sont rendus solidaires, soit de manière permanente (ou quasi-permanente) mais également parfois qu'une liaison est réalisée pour l'actionnement d'un élément par un autre. Ainsi, un vissage ou une coopération de forme pour verrouiller provisoirement les éléments entre sont couverts par ce terme non limitatif.

Les termes bague, manchon, ou tube désignent des structures creuses telles que des anneaux, des conduits ou des tuyaux mais de façon non limitative, notamment avec des formes diverses (à l'intérieur comme à l'extérieur) bien que la forme cylindrique soit préférée. Le terme canal est en revanche de préférence utilisé ici pour désigner un passage plutôt que l'élément qui le contient et le terme ouverture désigne ici le fait qu'un élément soit ouvert et apte à être traversé en débouchant dans une autre structure ou un autre élément. Généralement les termes manchon et tubes ou conduits désignent des éléments plus longs que les bagues ou anneaux, mais leur utilisation ici n'est pas limitative non plus. D'autre part, les termes douille ou culot désignent également des structures creuses qui sont ouvertes à une extrémité mais fermées à l'autre extrémité, comme des bouchons, des fermetures, des constrictions ou étranglement et ces termes sont utilisés indifféremment sans limitation quelconque.

Le terme « charnière » est utilisé ici dans son acception fonctionnelle, sans impliquer de limitation structurelle et peut désigner en fait des charnières mécaniques, même si elles sont de préférence formées (comme illustrés sur les exemples non limitatifs des figures), par des amincissement (ou rétrécissement, retrait de matière) des éléments tels que les bras de support ou autre. Ainsi, une charnière est en fait un point ou une zone d'articulation, puisqu'il est connu dans le domaine qu'il est généralement sans danger de prévoir de tels mécanismes de pivotement pour des implants car les matériaux qui les composent sont adaptés à ce type d'articulation. On notera que des amincissements des plateaux sont envisageables aussi, notamment à côté des articulations (charnières) des bras support, pour permettre au plateau de suivre la forme morphologique souhaitée, notamment lorsque les bras support n'ont pas les mêmes longueurs entre eux. Les termes « cylindre », « cylindrique » ou « cylindre généralisé » sont utilisés dans la présente demande sans distinction pour faciliter l'exposé de l'invention et désignent en fait tous un « cylindre généralisé » c'est-à-dire une forme tridimensionnelle définie par une hauteur (parallèle à l'axe longitudinal) et deux bases (transversales à l'axe longitudinal) qui peuvent avoir n'importe quelle forme, même si la forme circulaire est préférée pour simplifier la fabrication et limiter le risque de lésion des tissus dans lesquels elle est introduite. De préférence, ce « cylindre » est droit, c'est-à-dire que ses bases sont alignées selon la génératrice (ou hauteur) du cylindre. D'autre part, comme l'implant peut se déployer dans un tissu en se conformant à la forme de l'espace dans lequel il est introduit (en le modifiant grâce à la pression qu'il exerce sur ces issus), la forme peut ne pas être constante et les deux bases du cylindre peuvent avoir des formes (surfaces) différentes.

Par conséquent, le terme « diamètre » est utilisé, dans la présente demande, pour désigner en fait la plus grande dimension du cylindre généralisé transversalement à la hauteur (ou axe longitudinal), c'est-à-dire dans un plan (dit « transversal ») parallèle à celui des bases d'un tel cylindre généralisé. Ainsi, le terme diamètre peut en fait désigner la longueur de la diagonale d'un carré ou d'un rectangle ou encore (pour une forme quelconque) la plus grande distance entre deux points inclus dans un tel plan transversal et situés sur la circonférence d'un tel cylindre. De même, les termes « circonférence », « périphérie » ou « périmètre » sont utilisés ici pour désigner le pourtour de ces bases de forme quelconque.

De même, les termes « conique » ou « tronconique » sont utilisés ici pour désigner des formes qui s'évasent depuis un « diamètre » (ou une aire/surface) minimum jusqu'à un « diamètre » maximum, mais ils n'impliquent aucune limitation sur la forme de la périphérie qui peut être circulaire ou non.

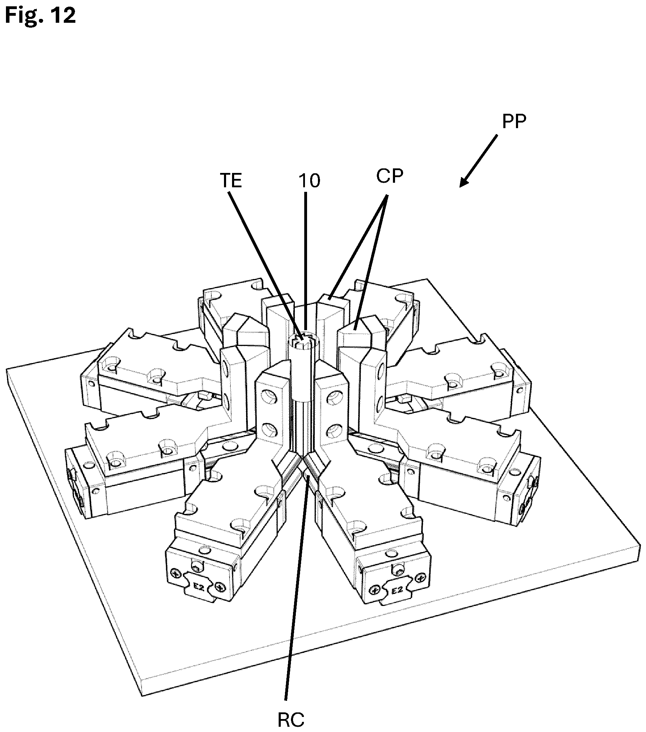

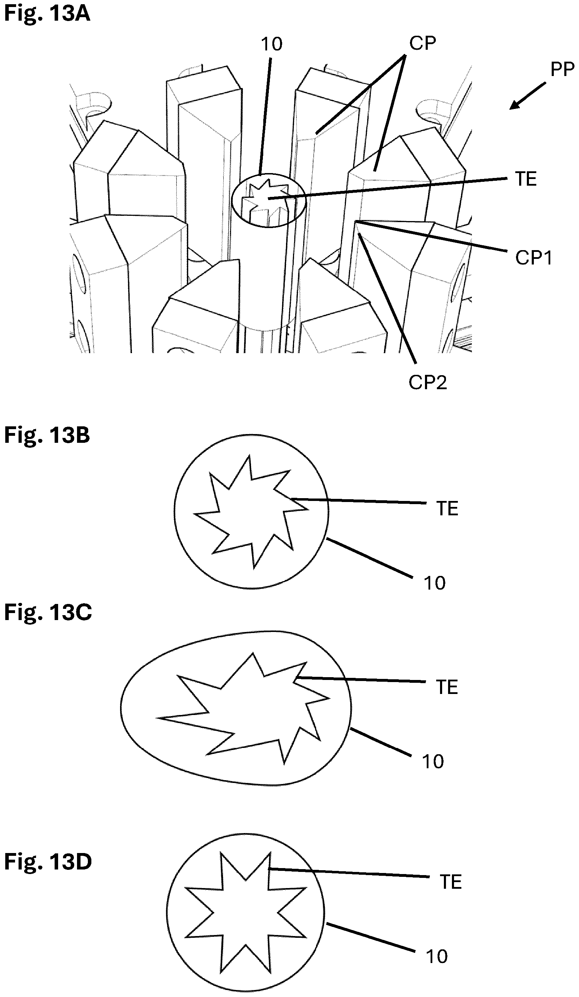

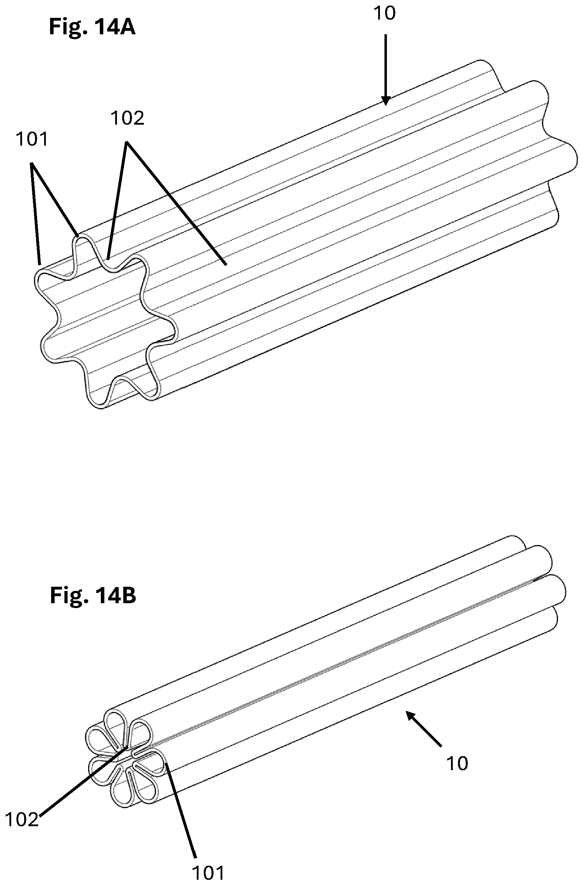

Les termes pli antiforme, dit convexe, et un pli synforme, dit concave son utilisés par analogie avec les définitions de plis dans de nombreux domaines techniques, dont la géologie, mais on comprendra que la convexité est ici définie par rapport à l'extérieur de l'implant. Un pli antiforme ou convexe est donc un pli qui rabat la matière vers l'intérieur, tandis qu'un plis antiforme rabat la matière vers l'extérieur. La succession des deux types de plis permet de limiter un maximum le volume replié. De plus, certains modes de réalisation prévoient des plis longs et des plis courts se succédant pour faciliter l'enroulement et/ou le tassement, en limitant la superposition de matière en configuration repliée. Pour faciliter l'enroulement de la feuille (10) sur elle-même et obtenir un volume replié moins important, il est préférable de prévoir une alternance de plis longs et de plis courts. Pour cela, il est possible d'utiliser des cames (CP) de pré-pliage comportant deux bords avec des angles différents, avec une tige étoilée (TE) possédant également une forme asymétrique et complémentaire du premier angle (CP1) de la came de pré-pliage et du deuxième angle (CP2) de la came de pré-pliage, comme par exemple représenté sur les figures 12, 13A, 13B et 13C mais il est possible également d'avoir une forme symétrique de pré-pliage, comme par exemple représenté sur les figures 13D, 14A et 14B, même si ces modes de réalisation permettent un repliement moins avantageux qu'un pliage asymétrique avec une alternance de plis longs et courts. On notera également que le nombre de plis n'est pas limitatif non plus et qu'il permet en revanche de conserver l'irrégularité ou la réalité de forme de l'implant lors du déploiement, ce qui présente également des avantages, notamment en termes de stabilisation. De plus, il reste préférable de prévoir un équi-répartition des surfaces entre les plis pour un déploiement uniforme permettre un déploiement uniforme mais l'invention envisage également d'autres applications et notamment des plis de différentes tailles selon la région de l'implant, afin d'obtenir un déploiement asymétrique et de meilleurs résultats thérapeutique. Par ailleurs, la présente invention permet de maîtriser la forme de l'implant une fois déployé en prévoyant également la distance entre les plis. En effet, la distance entre les plis synforme/antiforme et donc la distance entre les plis longs et les plis courts conditionne la façon de se déployer. Avantageusement, si la densité est plus dense à un endroit de la périphérie, le déploiement sera plus important et si elle est moins dense, l'enveloppe pourra moins se déployer. On comprend que l'obtient ainsi une asymétrie et une incurvation par un déploiement plus étendu dans les zones présentant le plus de plis. De même, il est possible de prévoir plus de matière (une grande surface de la feuille d'un côté par exemple, pour que l'expansion latérale soit plus importante de ce côté que de l'autre. Par ailleurs, dans certains modes de réalisation, la feuille est soudée aux plateaux et ne peut donc pas se déployer plus loin que la distance entre les plateaux, qui aura été réglé par le mécanisme de levée. On obtient ainsi un implant dont l'expansion est limitée dans une dimension (la dimension essentielle en générale, où l'on souhaite restaurer une hauteur ou largeur précise), mais pas dans une autre dimension, de sorte que l'injection de ciment déploiera l'enveloppe dans les volumes d faible densité osseuse éventuellement présentes autour de l'implant. On notera d'ailleurs que l'instrument d'injection de fluide (Ac) peut être muni de moyens de contrôler la pression injectée (un manomètre par exemple) et de savoir quel est le volume résultant, afin de contrôler efficacement l'expansion dans les tissus osseux.

Enfin, on comprend que l'instrumentation proposée dans la présente demande dans certains modes de réalisation, en utilisant un porte-implant (ou ancillaire) relativement classique pour tenir l'implant et l'introduire dans les tissus osseux, mais également moins classique pour l'expandre dans les tissus osseux, présente également l'avantage de pouvoir réaliser toutes les étapes d'implantation et de stabilisation avec un seul instrument et en une opération continue. En effet, l'ancillaire avec un tube creux d'acheminement du ciment au travers du tube qui retient le ciment, permet de disposer d'un instrument permettant d'effectuer l'opération chirurgicale rapidement et efficacement. Après le perçage, on introduit l'implant et sans retirer l'instrument, on peut gonfler l'enveloppe avec le ciment et ensuite retirer l'outil avant, pendant ou même après la polymérisation du ciment (par exemple à l'aide d'un mécanisme de coupe du ciment dur lors d'une rotation de l'instrument). Le temps d l'opération chirurgicale est bien entendu nettement diminué mais également la stabilité de l'implant qui n'est lâché à aucun moment tant qu'il n'est pas stabilisé par l'injection de ciment remplissant tous les volumes libres autour, contrairement à certaines solutions de l'art antérieur.

D'une manière générale, la présente demande concerne un implant (1) osseux expansible de chirurgie orthopédique humaine pour la restauration de volume et/ou géométrie d'un os, par une expansion entre une configuration repliée et une configuration déployée, ledit implant comportant un axe central (3) et s'étendant selon un axe longitudinal (L) entre une extrémité proximale (11) apte à coopérer avec un instrument d'implantation (A) pour tenir l'implant et une extrémité distale (12) destinée à être insérée en premier dans l'os, au moins deux faces, par exemple supérieure et inférieure, de l'implant comportant chacune au moins un plateau (13, 14, 15) pour le contact avec les tissus osseux, chacun des plateaux étant supportés par au moins deux bras de support (131, 141, 151) chacun, par l'intermédiaire d'une charnière sur l'axe central (3) et d'une charnière sous le plateau (13, 14, 15) respectif de chacun desdits bras de support (131, 141, 151) ;
caractérisé en ce que :
- une douille ou bague d'expansion (20) disposée dans le même axe que ledit axe central (3) ;
- au moins deux bras d'expansion (132, 142, 152) comportent chacun une charnière les reliant à l'une des extrémités de l'un des plateaux (13, 14, 15) et une charnière les reliant à ladite douille ou bague d'expansion (20) ;
- ladite douille ou bague d'expansion (20) et l'extrémité proximale de l'axe central (3) sont aptes à coopérer, respectivement ou inversement, avec un tube creux (A1) de préhension de l'implant (1) d'un instrument d'implantation (A) et avec une tige d'expansion (A3) dudit instrument (A) ;
- ladite tige d'expansion (A3) est apte à coulisser à l'intérieur dudit tube creux (A1), de façon à permettre de provoquer un éloignement entre ladite douille (3) et ledit axe central (3), exerçant une traction sur les plateaux (13, 14, 15), par l'intermédiaire des bras d'expansion (132, 142, 152), ce qui engendre un pivotement desdits bras de support (131, 141, 151) provoquant l'éloignement des plateaux (13, 14, 15) par rapport à l'axe central (3), de manière à résulter en une expansion contrôlée de l'implant (1) entre ladite configuration repliée et ladite configuration déployée.

Dans certains modes de réalisation, c'est ladite bague d'expansion (20) qui est apte à coopérer avec un tube creux (A1) de préhension de l'implant (1) dudit instrument d'implantation (A), en laissant passer la tige d'expansion (A3) à travers la bague, tandis que l'axe central (3) est apte à coopérer avec ladite tige d'expansion (A3) dudit instrument (A), de sorte qu'une poussée exercée sur ladite tige d'expansion (A3) coulissant à l'intérieur dudit tube creux (A1) provoque l'éloignement dudit axe central (3) par rapport à ladite bague (20), résultant en une expansion de l'implant (1) contrôlée en fonction de la force de poussée exercée sur la tige d'expansion (A3).

Dans certains modes de réalisation alternatifs des précédents, c'est l'extrémité proximale de l'axe central (3) qui est apte à coopérer avec ledit tube creux (A1) de préhension de l'implant (1) dudit instrument d'implantation (A), tandis que ladite douille ou bague d'expansion (20) est apte à coopérer avec ladite tige d'expansion (A3) dudit instrument (A), de sorte qu'une poussée exercée sur ladite tige d'expansion (A3) coulissant à l'intérieur dudit tube creux (A1) provoque l'éloignement de ladite douille ou bague d'expansion (20) par rapport audit axe central (3), résultant en une expansion de l'implant (1) contrôlée en fonction de la force de poussée exercée sur la tige d'expansion (A3).

Dans certains modes de réalisation, l'axe central (3) comporte un conduit (31) apte à coopérer avec ladite tige d'expansion (A3) qui est, d'une part, creuse et munie d'au moins une ouverture au niveau de son extrémité distale et, d'autre part, connectable à un instrument (AC) d'injection de fluide pour acheminer au moins un fluide dans le site d'implantation de l'implant via l'axe central (3), de préférence muni d'ouvertures (32) pour faire déboucher le fluide dans la longueur de l'axe central (3).

Dans certains modes de réalisation, les bras de support (131, 141, 151) des plateaux comportent deux bras reliés respectivement à proximité des extrémités proximale et distale de leur plateau (13, 14, 15) respectif, pour fournir un support sur toute la longueur des plateaux et limiter leurs risques de cintrage ou fluage.

Implant selon l'une des revendications précédentes, caractérisé en ce qu'au moins un bras de support central (130, 140, 150) supplémentaire est relié entre une portion centrale de l'axe central et une portion centrale des plateaux (13, 14, 15), avec des charnières aux deux extrémités du bras de support (130, 140, 150) pour son pivotement.

Dans certains modes de réalisation, deux plateaux (13, 14) disposés de part et d'autre de l'axe central (3) pour fournir un appui contre des tissus osseux lésés de part et d'autre de l'implant, par exemple pour restaurer une hauteur ou une largeur.

Dans certains modes de réalisation, l'implant comporte au moins un plateau (15) additionnel, la répartition des plateaux autour de l'axe central (3) variant en fonction de leur nombre et/ou des besoins en termes de traitement chirurgical, avec de préférence une équi-répartition angulaire radialement par rapport à l'axe central, pour exercer une compression homogène sur les tissus osseux à la périphérie de l'implant.

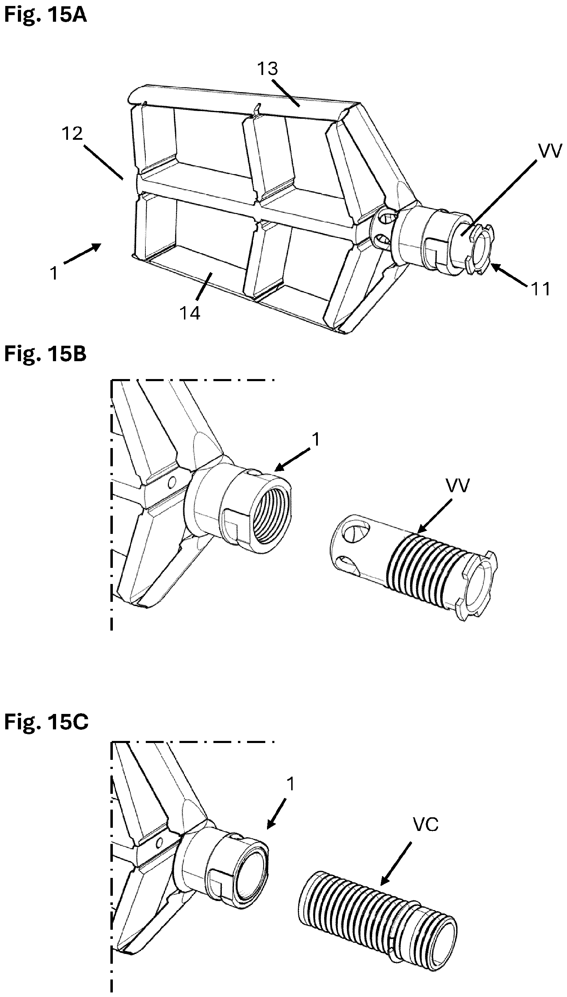

Dans certains modes de réalisation, des bras support en doubles exemplaires sont prévus pour renforcer la structure. D'autre part, des moyens de verrouillage sont prévus dans certains cas pour empêcher l'implant de se replier. Les diamètres très fins des implants et leurs axes centraux sont difficilement compatibles avec des filetages pour opérer l'expansion par vissage au niveau de l'implant, alors qu'il est avantageux d'effectuer un vissage au niveau de l'instrument actionnant l'expansion, notamment lorsque l'expansion implique un rapprochement des bras support les uns par rapport aux autres. Ainsi, comme connu de l'art antérieur, il est possible d'utiliser par exemple une bague fendue logée dans un renforcement circulaire de l'implant et coopérant avec des crans sur l'axe de poussée ou de traction qui sont orientés pour permettre le passage de cet axe dans un seul sens, par exemple comme représenté sur les figures 15C. Ainsi, l'actionnement de l'axe pour l'expansion des plateaux peut être réalisé par passage successifs des crans, ce qui permet de verrouiller l'implant en configuration déployée.

Cependant, contrairement à certains implants de l'art antérieur où l'axe de traction permettant de rapprocher doit nécessairement rester en place, les implants de la présente demande sont déployés sans rapprochement des bras de support, ce qui permet avantageusement de pouvoir les verrouiller avec un verrou vissé et de ne pas nécessiter de tels crans rendant la tâche difficile et offrant une fiabilité réduite. Ainsi, par exemple comme représenté sur les figures 15A et 15B, il est possible d'utiliser par exemple un manchon fileté configuré pour être disposé dans le tube creux de l'instrument d'implantation (A) tenant l'implant (et entourer l'éventuel conduit d'injection de fluide présent à l'intérieur). Un tel manchon possède alors un filetage prévu pour coopérer avec un taraudage de l'extrémité proximale (11) de l'implant et présente des moyens d'actionnement pour le vissage ou dévissage (comme des ailettes radiales représentées sur la figure 15B).

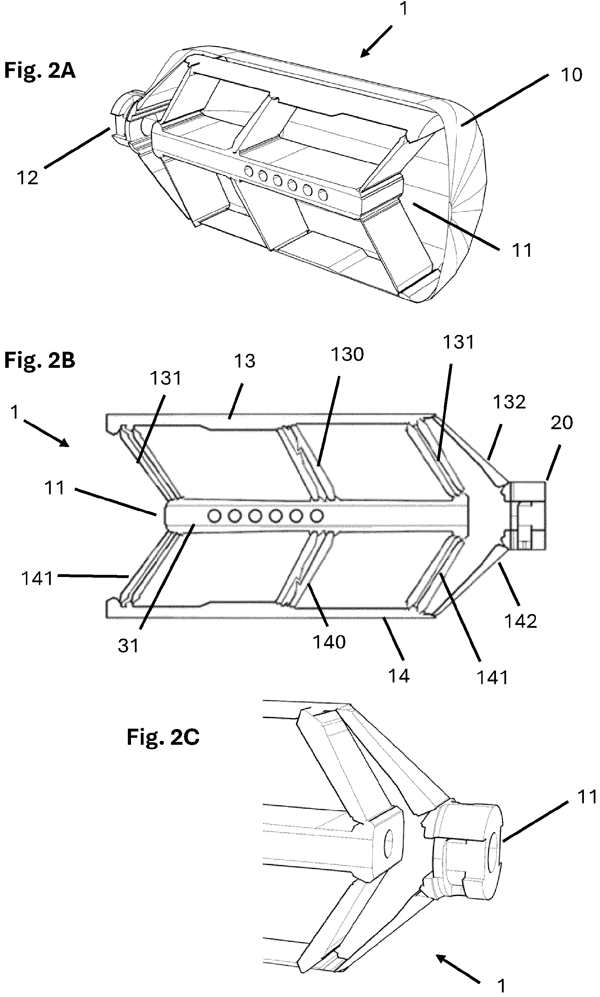

De plus, l'agencement des implants de la présente demande fournit un avantage non négligeable en ce qui concerne la fiabilité de l'expansion. En effet, par le fait que les bras support sont disposés au moins aux extrémités des plateaux (et éventuellement avec un ou plusieurs bras de renfort entre les extrémités) permet de créer des parallélogrammes déformables qui conservent leur propriété de parallélisme entre leurs côtés, contrairement à certains implants de l'art antérieur où les bras de support sont montés en opposition. Ce type d'implants de l'art antérieur nécessitent généralement que les bras soient prévus en doubles exemplaires de chaque côté pour améliorer la fiabilité de l'expansion. Les implants de la présente demande ne nécessitent pas de doubler les bras, mais il reste cependant possible de le faire, notamment dans le cas d'implants de grande taille et/ou destinés à supporter une charge importante. Ainsi, certains modes de réalisation comportent des bras supports en double exemplaire, au moins à l'une des positions de ces bras et de préférence à chacun d'entre elles, par exemple comme représenté sur la figure 2B. De plus, de tels bras doubles peuvent comporter un mécanisme d'auto-verrouillage en configuration déployée, comme par exemples des crans ménagés face à face de sorte à s'engager entre eux, par exemple comme représenté pour les bras de support central (130, 140) de la figure 2B.

La présente demande concerne aussi un système de traitement orthopédique de tissu osseux lésé comprenant un ciment de substitution osseuse et au moins un instrument d'implantation (A) et d'injection de ciment (Ac) dans l'implant (1), caractérisé en ce qu'il comporte un implant (1) selon divers modes de réalisation.

Dans certains modes de réalisation, l'instrument d'implantation (A) et d'injection de ciment (Ac) comporte des moyens de contrôle de la pression et/ou de l'aspiration du ciment pour replier l'implant en configuration repliée si nécessaire.

Dans certains modes de réalisation, l'instrument d'implantation (A) est distinct mais complémentaire de l'instrument d'injection (Ac) dont le canal d'injection du ciment traverse un canal à l'intérieur de la tige de l'instrument d'implantation (A) tenant l'extrémité proximale de l'implant (1) grâce à son extrémité distale.

Dans certains modes de réalisation, l'implant peut comporter une seconde feuille (10) entourant la première feuille, dans le même matériau ou un autre, par exemple pour une isolation thermique protégeant les tissus de la chaleur de la polymérisation. Dans ce cas, les extrémités comportent une bague supplémentaire pour fixer cette seconde feuille en préservant un espace entre celle-ci et la première feuille, avec potentiellement une entrée d'injection d'un autre fluide entre les deux. Ces deux feuilles peuvent alors être pliés et enroulées en même temps lors de la fabrication. Ces modes de réalisation permettent un préformage du site d'injection (par écrasement du tissus osseux spongieux) et permet par exemple d'injecter en deux temps ledit fluide pour un meilleur ajustement de la forme, de la température résultante dans les tissus et/ou de la vitesse de polymérisation du fluide.

Certains modes de réalisation concernent également un procédé de fabrication d'un implant selon l'une des revendications précédentes, caractérisé en ce qu'il comporte :
- Obtention d'un implant expansible à deux plateaux s'écartant sous l'effet du rapprochement des extrémités de l'implant grâce à des bras supports reliant ces extrémités aux plateaux ;
- Fermeture de la feuille sur elle-même et soudure pour former un cylindre généralisé ;
- Insertion de la feuille refermée sur une matrice en forme de cylindre généralisé possédant une base en forme d'étoile, dite tige étoilée (TE), le nombre de branches de l'étoile définissant le nombre de paires de plis de ladite feuille dudit implant ;
- Compression de la feuille refermée entre ladite matrice et une pluralité d'éléments saillants de forme complémentaire aux creux entre les branches de l'étoile ;
- Enroulement des plis de ladite feuille autour de l'axe longitudinal,
- Insertion dudit implant expansible à l'intérieur de ladite feuille compressée ;
- Solidarisation de ladite feuille sur la douille et le manchon.

Par exemple l'enroulement peut être réalisé par l'introduction de la feuille refermée et pré-pliée dans un conduit dont le diamètre se rétrécit progressivement jusqu' au diamètre souhaité pour l'implant, par coulissement et rotation de l'implant dans ce conduit (par exemple avec un guide à l'intérieur de la feuille pour éviter qu'elle ne s'écrase).

La solidarisation avec le manchon et la douille se fera généralement par soudure (120) et de préférence avec un écrasement préalable de la feuille sur la circonférence de la douille ou le manchon, par exemple par une bague de compression (121) dont des exemples sont représentés sur certaines figures. En effet, il est possible de souder directement mais un écrasement des plis à l'endroit reste préférable.

La présente demande décrit diverses caractéristiques techniques et avantages en référence aux figures et/ou à divers modes de réalisation. L'homme de métier comprendra que les caractéristiques techniques d'un mode de réalisation donné peuvent en fait être combinées avec des caractéristiques d'un autre mode de réalisation à moins que l'inverse ne soit explicitement mentionné ou qu'il ne soit évident que ces caractéristiques sont incompatibles ou que la combinaison ne fournisse pas une solution à au moins un des problèmes techniques mentionnés dans la présente demande. De plus, les caractéristiques techniques décrites dans un mode de réalisation donné peuvent être isolées des autres caractéristiques de ce mode à moins que l'inverse ne soit explicitement mentionné.

### Liste détaillée des références dans les figures :

- 1: implant
- 10: feuille
- 11: extrémité proximale
- 101: pli antiforme
- 102: pli synforme
- 110: soudure proximale
- 12: extrémité distale
- 120: soudure distale (liaison étanche)
- 121: fixation par compression (e.g., bague fendue)
- 3: axe central
- 31: conduit dans l'axe central
- 32: ouvertures du conduit de l'axe central
- 13: premier plateau
- 14: deuxième plateau
- 15: troisième plateau
- 20: bague d'expansion
- 131: bras support du premier plateau
- 141: bras support du deuxième plateau
- 151: bras support du troisième plateau
- 132: bras d'expansion du premier plateau
- 142: bras d'expansion du deuxième plateau
- 152: bras d'expansion du troisième plateau
- 130: bras support central du premier plateau
- 140: bras support central du deuxième plateau
- 150: bras support central du troisième plateau
- A: instrument d'implantation
- Ac: instrument d'injection de fluide
- A1: tube creux de préhension
- A3: tige d'expansion ?
- TG: tige de guidage
- GR: guide de recouvrement
- PP: Platine de pré-pliage
- ET: tige étoilée
- CP: came de pré-pliage
- CP1: premier angle de came de pré-pliage
- CP2: deuxième angle de came de pré-pliage
- RC: rampe de came
- VV: verrou vissé
- VC: verrou cranté

## Revendications

1. Implant (1) osseux expansible de chirurgie orthopédique humaine pour la restauration de volume et/ou géométrie d'un os, par une expansion entre une configuration repliée et une configuration déployée, ledit implant comportant un axe central (3) et s'étendant selon un axe longitudinal (L) entre une extrémité proximale (11) apte à coopérer avec un instrument d'implantation (A) pour tenir l'implant et une extrémité distale (12) destinée à être insérée en premier dans l'os, au moins deux faces, par exemple supérieure et inférieure, de l'implant comportant chacune au moins un plateau (13, 14, 15) pour le contact avec les tissus osseux, chacun des plateaux étant supportés par au moins deux bras de support (131, 141, 151) chacun, par l'intermédiaire d'une charnière sur l'axe central (3) et d'une charnière sous le plateau (13, 14, 15) respectif de chacun desdits bras de support (131, 141, 151) ; **caractérisé en ce que :**
- une douille ou bague d'expansion (20) disposée dans le même axe que ledit axe central (3) ;
- au moins deux bras d'expansion (132, 142, 152) comportent chacun une charnière les reliant à l'une des extrémités de l'un des plateaux (13, 14, 15) et une charnière les reliant à ladite douille ou bague d'expansion (20) ;
- ladite douille ou bague d'expansion (20) et l'extrémité proximale de l'axe central (3) sont aptes à coopérer, respectivement ou inversement, avec un tube creux (A1) de préhension de l'implant (1) d'un instrument d'implantation (A) et avec une tige d'expansion (A3) dudit instrument (A) ;
- ladite tige d'expansion (A3) est apte à coulisser à l'intérieur dudit tube creux (A1), de façon à permettre de provoquer un éloignement entre ladite douille (3) et ledit axe central (3), exerçant une traction sur les plateaux (13, 14, 15), par l'intermédiaire des bras d'expansion (132, 142, 152), ce qui engendre un pivotement desdits bras de support (131, 141, 151) provoquant l'éloignement des plateaux (13, 14, 15) par rapport à l'axe central (3), de manière à résulter en une expansion contrôlée de l'implant (1) entre ladite configuration repliée et ladite configuration déployée ;
**et en ce qu'**il comporte une enveloppe renfermant ledit implant depuis l'extrémité proximale (11) jusqu'à l'extrémité distale (12) et **en ce que** :
- ladite enveloppe est formée par une feuille (10) en alliage biocompatible de métaux, refermée sur elle-même de manière étanche ;
- ladite feuille (10) présente, au moins dans la configuration repliée, une pluralité de paires de plis, chacune des paires comportant un pli antiforme (101), dit convexe, et un pli synforme (102), dit concave, lesdits plis étant couchés les uns sur les autres en configuration repliée de sorte que les surfaces présentes entre chacun desdits plis convexes et concaves soient enroulées autour de l'axe longitudinal (L) ;
- ladite extrémité proximale (11) se prolonge par un manchon d'étanchéité solidarisé de manière étanche, aux plis couchés et enroulés de ladite feuille (10) sur toute la périphérie de l'extrémité proximale (11) ;
- ladite extrémité distale (12) se prolonge par une douille solidarisé, de manière étanche, aux plis couchés et enroulés de ladite feuille (10) sur toute la périphérie de l'extrémité distale (12) dudit implant (1) ;
- ladite feuille (10) est déformable plastiquement pour permettre l'expansion de l'implant de la configuration repliée à la configuration déployée en formant une enveloppe étanche enfermant l'implant et permettant d'éviter les fuites lors de l'injection d'un fluide à l'intérieur de l'implant (1) et l'enveloppe.

2. Implant selon la revendication 1, **caractérisé en ce que** c'est ladite bague d'expansion (20) qui est apte à coopérer avec un tube creux (A1) de préhension de l'implant (1) dudit instrument d'implantation (A), en laissant passer la tige d'expansion (A3) à travers la bague, tandis que l'axe central (3) est apte à coopérer avec ladite tige d'expansion (A3) dudit instrument (A), de sorte qu'une poussée exercée sur ladite tige d'expansion (A3) coulissant à l'intérieur dudit tube creux (A1) provoque l'éloignement dudit axe central (3) par rapport à ladite bague (20), résultant en une expansion de l'implant (1) contrôlée en fonction de la force de poussée exercée sur la tige d'expansion (A3).

3. Implant selon la revendication 1, **caractérisé en ce que** c'est l'extrémité proximale de l'axe central (3) qui est apte à coopérer avec ledit tube creux (A1) de préhension de l'implant (1) dudit instrument d'implantation (A), tandis que ladite douille ou bague d'expansion (20) est apte à coopérer avec ladite tige d'expansion (A3) dudit instrument (A), de sorte qu'une poussée exercée sur ladite tige d'expansion (A3) coulissant à l'intérieur dudit tube creux (A1) provoque l'éloignement de ladite douille ou bague d'expansion (20) par rapport audit axe central (3), résultant en une expansion de l'implant (1) contrôlée en fonction de la force de poussée exercée sur la tige d'expansion (A3).

4. Implant selon l'une des revendications précédentes, **caractérisé en ce que** l'axe central (3) comporte un conduit (31) apte à coopérer avec ladite tige d'expansion (A3) qui est, d'une part, creuse et munie d'au moins une ouverture au niveau de son extrémité distale et, d'autre part, connectable à un instrument (AC) d'injection de fluide pour acheminer au moins un fluide dans le site d'implantation de l'implant via l'axe central (3), de préférence muni d'ouvertures (32) pour faire déboucher le fluide dans la longueur de l'axe central (3).

5. Implant selon l'une des revendications précédentes, **caractérisé en ce que** les bras de support (131, 141, 151) des plateaux comportent deux bras reliés respectivement à proximité des extrémités proximale et distale de leur plateau (13, 14, 15) respectif, pour fournir un support sur toute la longueur des plateaux et limiter leurs risques de cintrage ou fluage.

6. Implant selon l'une des revendications précédentes, **caractérisé en ce qu'**au moins un bras de support central (130, 140, 150) supplémentaire est relié entre une portion centrale de l'axe central et une portion centrale des plateaux (13, 14, 15), avec des charnières aux deux extrémités du bras de support (130, 140, 150) pour son pivotement.

7. Implant selon l'une des revendications précédentes, **caractérisé en ce qu'**il comporte deux plateaux (13, 14) disposés de part et d'autre de l'axe central (3) pour fournir un appui contre des tissus osseux lésés de part et d'autre de l'implant, par exemple pour restaurer une hauteur ou une largeur.

8. Implant selon l'une des revendications 1 à 6, **caractérisé en ce qu'**il comporte au moins un plateau (15) additionnel, la répartition des plateaux autour de l'axe central (3) variant en fonction de leur nombre et/ou des besoins en termes de traitement chirurgical, avec de préférence une équi-répartition angulaire radialement par rapport à l'axe central, pour exercer une compression homogène sur les tissus osseux à la périphérie de l'implant.

9. Implant selon l'une des revendications précédentes, **caractérisé en ce que** la distance entre un pli synforme et le pli antiforme suivant est plus longue que la distance entre un pli antiforme et le pli synforme suivant, pour faciliter l'enroulement des plis autour de l'axe longitudinal (L) de l'implant en configuration repliée.

10. Implant selon l'une des revendications précédentes, **caractérisé en ce que** ledit manchon d'étanchéité prolonge ladite extrémité proximale (11), parallèlement à l'axe longitudinal (L), jusqu'à une distance qui est supérieure ou égale à celle à laquelle s'étendent les plateaux depuis le centre de l'implant (1)

11. Implant selon l'une des revendications précédentes, **caractérisé en ce que** ledit manchon d'étanchéité possède une ouverture traversante dont le diamètre est supérieur ou égal à celui de l'ouverture du manchon de coulissement, de sorte que ces deux manchons d'étanchéité et de coulissement fournissent une entrée vers l'intérieur du corps creux de l'implant (1) depuis un conduit d'un instrument (A) d'implantation tenant l'implant au niveau de l'extrémité proximale, apte à acheminer un fluide à injecter dans ledit implant.

12. Implant selon l'une des revendications précédentes, **caractérisé en ce qu'**il comporte, en position déployée, une portion médiane entre ses deux extrémités qui possède une forme de cylindre généralisé de longueur supérieure ou égale à celle des plateaux, avec une persistance éventuelle et partielle desdits plis, ladite portion médiane se prolongeant, du côté de l'extrémité proximale (11), par une portion tronconique reliant la portion médiane au manchon et, du côté de l'extrémité distale (12), par une portion tronconique reliant la portion médiane à la douille, les portions tronconiques présentant une persistance permanente d'au moins une partie de plis couchés et enroulés à proximité des extrémités proximale (11) et distale (12).

13. Implant selon l'une des revendications précédentes, **caractérisé en ce que** ladite feuille est déformable plastiquement également de la configuration repliée à la configuration déployée, notamment grâce à la persistance des plis couchés et enroulés aux extrémités proximale et distale, facilitant la réversibilité de l'expansion.

14. Implant selon l'une des revendications précédentes, **caractérisé en ce que** ledit axe central (3) est apte à coopérer avec et/ou se prolonge au-delà de l'extrémité distale d'un instrument (A) d'implantation au niveau de l'extrémité proximale de l'implant et possédant un conduit intérieur en communication avec un conduit (31) ménagé dans ledit axe central débouchant dans l'espace ménagé par l'écartement des plateaux, via au moins une ouverture (32) permettant l'injection dudit fluide dans l'implant (1).

15. Implant selon l'une des revendications précédentes, **caractérisé en ce que** ladite feuille (10) est solidarisée à l'extrémité proximale (11) par une soudure (110) figeant l'extrémité proximale des plis couchés et enroulés contre la paroi extérieure dudit manchon de coulissement et/ou solidarisée à l'extrémité distale (12) par une soudure (120) figeant l'extrémité distale des plis couchés et enroulés contre la paroi extérieure de ladite douille de traction.

16. Implant selon l'une des revendications précédentes, **caractérisé en ce que** ladite feuille est comprimée autour du manchon de l'extrémité proximale (11) et/ou autour de la douille de l'extrémité distale (12) par une bague de compression (121) maintenant les plis couchés et enroulés contre la paroi extérieure dudit manchon et/ou de ladite douille.

17. Implant selon l'une des revendications précédentes, **caractérisé en ce que** les plis sont, au moins dans la configuration repliée, parallèles à l'axe longitudinal (L).

18. Implant selon l'une des revendications précédentes, **caractérisé en ce que** la feuille comporte également au moins une paire de plis (un pli synforme et un pli antiforme) d'axe non parallèle à l'axe longitudinal (L), de préférence perpendiculaire pour une expansion également en longueur de l'implant ou oblique pour une expansion incurvée de l'implant.

19. Implant selon l'une des revendications précédentes, **caractérisé en ce que** le nombre de paires de plis est compris entre 3 et 16, généralement 4 à 12, de préférence de l'ordre de 8.

20. Implant selon l'une des revendications précédentes, **caractérisé en ce que** la feuille (10) possède une épaisseur comprise entre 3 et 100 microns, généralement entre 6 et 50 et de préférence 10 et 30 microns.

21. Implant selon l'une des revendications précédentes, **caractérisé en ce que** la feuille (10) est en alliage de titane.

22. Implant selon l'une des revendications précédentes, **caractérisé en ce que** la distance entre les plis est variable d'une face latérale à l'autre de l'implant, de sorte que la forme de l'implant en configuration déployée soit incurvée et/ou asymétrique transversalement à l'axe longitudinal (L).

23. Système de traitement orthopédique de tissu osseux lésé comprenant un ciment de substitution osseuse et au moins un instrument (A) d'implantation et d'injection (Ac) de ciment dans l'implant (1), **caractérisé en ce qu'**il comporte un implant (1) selon l'une des revendications précédentes.

24. Système selon la revendication 23, **caractérisé en ce que** l'instrument d'implantation et d'injection de ciment comporte des moyens de contrôle de la pression et/ou de l'aspiration du ciment pour replier l'implant en configuration repliée si nécessaire.

25. Système selon l'une des revendications 23 et 24, **caractérisé en ce que** l'instrument d'implantation est distinct mais complémentaire de l'instrument d'injection dont le canal d'injection du ciment traverse un canal à l'intérieur de la tige de l'instrument d'implant tenant l'extrémité proximale de l'implant grâce à son extrémité distale.

26. Procédé de fabrication d'un implant selon l'une des revendications précédentes, **caractérisé en ce qu'**il comporte :
- Obtention d'un implant expansible à deux plateaux s'écartant sous l'effet du rapprochement des extrémités de l'implant grâce à des bras supports reliant ces extrémités aux plateaux ;
- Fermeture de la feuille sur elle-même et soudure pour former un cylindre généralisé ;
- Insertion de la feuille refermée sur une matrice en forme de cylindre généralisé possédant une base en forme d'étoile, dite tige étoilée (TE), le nombre de branches de l'étoile définissant le nombre de paires de plis de ladite feuille dudit implant ;
- Compression de la feuille refermée entre ladite matrice et une pluralité d'éléments saillants de forme complémentaire aux creux entre les branches de l' étoile ;
- Enroulement des plis de ladite feuille autour de l'axe longitudinal,
- Insertion dudit implant expansible à l'intérieur de ladite feuille compressée ;
- Solidarisation de ladite feuille sur la douille et le manchon.
